(19) Europäisches Patentamt
European Patent Office
Office européen des brevets

(11) **EP 1 738 683 A1**

(12) **EUROPEAN PATENT APPLICATION**

(43) Date of publication:
**03.01.2007 Bulletin 2007/01**

(51) Int Cl.:
**A61B 5/00** *(2006.01)* **G01K 11/00** *(2006.01)*
**G01J 5/60** *(2006.01)*

(21) Application number: **05405418.4**

(22) Date of filing: **30.06.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Pixartis SA**
**1005 Lausanne (CH)**

(72) Inventors:
• **Butz, Torsten**
**9052 Niederteufen (CH)**
• **Dati, Emanuele**
**1008 Prilly (CH)**

• **Escoda, Oscar Divorra**
**Princeton, NJ 08540 (US)**
• **Kunt, Murat**
**1603 Grandvaux (CH)**
• **Vandergheynst, Pierre**
**1009 Pully (CH)**

(74) Representative: **Ganguillet, Cyril**
**ABREMA**
**Agence Brevets & Marques**
**Ganguillet & Humphrey**
**Avenue du Théâtre 16**
**Case postale 5027**
**1002 Lausanne (CH)**

(54) **Microwave temperature image reconstruction**

(57) A multi-frequency spiral microwave antenna (1) of the microwave image reconstruction system senses a signal which passes through an analogue connection (6) to a Dicke null-balancing radiometer (7) which detects sequentially the signal contributions at the different frequencies of the multi-frequency antenna of the system. The resulting sequential analogue signals from the radiometer are directly related to the brightness temperatures at the corresponding frequencies, and these brightness temperatures are directly related to a real intra-body temperature distribution. The system uses a PC (5) and comprises a mean to reconstruct a one-dimensional profile of real intra-body temperatures from the brightness temperatures via the outputs from the analogue-to-digital converter (15). The algorithm of this system uses "large" redundant dictionaries which, on the one hand reflect the variability of potential temperature profiles and on the other hand incorporate as much a-priory information as possible, in order to provide reliable image reconstruction.

PC with drivers for data converter and reconstruction algorithm — 5, 9
PC data bus — 8
A/D-converter — 15
Null-balancing radiometer — 7
Analogue connection — 6
A multi-frequency spiral antenna — 1

**FIG.6**

EP 1 738 683 A1

**Description**

**Technical Field of the Invention**

The present invention relates to a microwave temperature image reconstruction system. More specifically, the present invention relates to the use of an algorithm which shows itself particularly adapted to temperature image reconstruction.

**Background of Microwave Imaging**

The large potential of temperature imaging has been recognized for long. For example in the medical community such data is known to potentially improve a variety of diagnostic and interventional procedures. In the case of industrial applications, thermal information has shown to predict and prevent failures of electrical circuits and equipment. One potentially interesting imaging modality for temperature imaging is microwave imaging. Due to several practical drawbacks, microwave imaging has hardly been able to enter the industrial or medical end-user market though. One of these drawbacks lies in the difficult image reconstruction task which contrasts with the rather simple data acquisition process of a potential microwave imaging system.

Non-Destructive Evaluation (NDE) has been one of the engineering disciplines which mostly revolutionized diagnostic techniques in industry and in medicine during the last decades. MR (Magnetic Resonance), CT (Computerized Tomography), US (Ultra-Sound), and other NDE devices are being standard tools for a wide range of diagnostic fields. Furthermore they are currently changing significantly medical surgery, as their capability of visualizing intra-body information enables surgeons to minimize the invasiveness of their interventions. Even though NDE techniques are by themselves expensive, they are potentially even interesting from a financial point of view, as they can significantly decrease the expensive hospitalization time of patients. Similar arguments apply to industrial NDE which was able to bring quality assurance and failure prediction to an impressive performance.

Several NDE modalities are currently being used, including MR, CT, and US imaging. Each of these modalities images a particular physical quantity, such as MR can image proton density, CT images x-ray absorption coefficients, or US visualizes reflection coefficients for ultrasound waves. Therefore each of these modalities has applications for which they are mostly adapted, while being excluded from other applications due to their incapability to image the appropriate physical quantity. As a result, the development of new NDE imaging modalities which are able to image additional physical quantities is a main branch of engineering and fundamental research. One of these quantities, which have been recognized to be particularly valuable for a wide range of medical and industrial applications, is intra-body temperature. Currently, just MR imaging is capable to perform such measurements, though being prohibitively expensive for a wide range of promising applications. As an example, cancer development is known to be accompanied with a local temperature increase of 1-2° C. Nevertheless, the temperature resolution and mere cost of MR temperature imaging prohibits its use in the important field of breast cancer detection.

As an alternative to MR temperature imaging, microwave temperature monitoring is a promising avenue. Microwave temperature imaging devices are in comparison to MR devices significantly smaller, cheaper, and less complex to implement. Nevertheless, their current research implementations are too premature to be produced and marketed on large scales. One of the main drawbacks lies in the fact that the data collected by microwave imaging devices is very sparse in comparison to the temperature resolution and spatial resolution one would like to obtain for the final temperature profiles.

**Summary of the Invention**

The present invention primarily proposes a solution to this reconstruction problem. To achieve this objective a microwave image reconstruction algorithm is disclosed which uses "large" redundant dictionaries which, on the one hand reflect the variability of potential temperature profiles and on the other hand incorporate as much a-priory information as possible, in order to provide reliable image reconstruction. Together with state-of-the-art algorithms for data decomposition over redundant dictionaries, temperature profiles can be reconstructed reliably.

The above and other objects, features and advantages of the present invention will become apparent from the following description when taken in conjunction with the accompanying drawings which illustrates preferred embodiments of the present invention by way of example.

**Brief Description of the Drawings**

Fig. 1 shows 3 B-splines of a set of 100 B-splines which cover regularly the imaging space and whose weighted sum constitutes the temperature profile reconstruction.

Fig. 2 shows three results of current state-of-the-art microwave temperature profile reconstruction by Tikhonov regularization and its limitations. The reference and reconstructed profiles are shown on the left side, while the resulting expansion coefficients are presented in the right column.

Fig. 3 shows the basic building blocks necessary for a microwave temperature monitoring device with a reconstruction procedure as the one disclosed herein.

Fig. 4 shows a schematic sketch of the functional building blocks necessary to realize the disclosed invention.

Fig. 5 shows the functional building blocks for data decomposition over redundant dictionaries as used in the disclosed invention.

Fig. 6 presents a sketch of the building blocks for a PC-based implementation of the disclosed invention.

Fig. 7 shows a more detailed view of the PC-based sample implementation of a microwave temperature imaging device.

Fig. 8 shows cubic B-splines at scale 0 and scale 9 covering regularly the imaging space. These functions were part of the basis function set, used in the sample implementations of the disclosed invention.

Fig. 9 presents three reconstruction examples for the PC-based sample device. On the left side, the reconstructed and reference profiles are drawn. On the right side, the determined optimal expansion coefficients are shown. These coefficients correspond to the reconstruction profiles shown on the left.

Fig. 10 shows a sketch of the functional building blocks for the embedded sample implementation of the disclosed invention.

Fig. 11 shows a more detailed view of the embedded sample implementation of a microwave temperature imaging device.

Fig. 12 shows the flow chart of the FOCUSS algorithm as used for the temperature profile image reconstruction on the embedded sample implementation of the disclosed invention.

Fig. 13 shows three reconstruction results for the embedded sample device. The reconstructed and reference profiles are drawn on the left side, while the optimal expansion coefficients are drawn on the right side.

**Detailed Description of the invention**

Microwave image reconstruction aims to solve the following equation for the unknown temperature profile, noted $T(\mathbf{x})$:

$$b_i = \gamma_i \cdot \int_{\Omega} W_i(\mathbf{x}) \cdot T(\mathbf{x}) d\mathbf{x}, \text{ with } i = 1, 2, ..., N \qquad \text{Eq. 1}$$

where $i$ indexes the $N$ measurements of the system at frequencies, $f_i$. $b_i$ are the measured brightness temperatures sensed by the multi-frequency imaging antenna at different frequencies $f_i$, as described in **[2, 9]**. $\gamma_i$ are the antenna efficiencies at frequencies $f_i$, $W_i(\mathbf{x})$ are the spatial weighting functions at frequencies $f_i$, and $\Omega$ is the sensing space of the microwave antenna. The spatial variable x may be a 1D, 2D, or 3D spatial vector, depending on the dimension of the reconstruction problem. In the 1D case, the number of measurements $N$ lies at around 5 to 7, i.e. the antenna senses the brightness temperatures $b_i$ at 5 to 7 different frequencies $f_i$. Therefore we have just 5 to 7 measurements to recover the whole 1D temperature profile $T(\mathbf{x})$.

The weighting function $W(f, \mathbf{x})$ weights the brightness temperature contributions from different spatial positions x. It therefore accounts for the absorption process of the temperature dependent thermal radiation from any point inside the investigated body to the measurement antenna.

Algorithms currently being used for the microwave image reconstruction of eq. 1 are presented in **[1, 2]**. Both approaches attempt to resolve the problem of having just very sparse radiometric data available for the reconstruction, by giving a very restrictive prior on the temperature profile shape one would like to recover. In **[1]** and in the case of a 1D profile, the profile is assumed to be a single Gaussian:

$$T(\mathbf{x}) = T_0 \cdot \exp\left(-\left(\frac{\mathbf{x} - \mathbf{x_o}}{\sigma}\right)\right), \qquad \text{Eq. 2}$$

with $T_0$ being the amplitude of the Gaussian, $\mathbf{x_0}$ its position and $\sigma$ its variance. The reconstruction algorithm determines the parameters, $T_0$, $\mathbf{x_0}$ and $\sigma$, which explain the best the measurements $b_i$ through eq. 1. The problem with this approach lies in the very strong Gaussian assumption of eq. 2. This assumption is too restrictive to deal with a large number of real world temperature profiles.

A more flexible approach to temperature image reconstruction has been proposed in **[2]**. There, a small sum of Chebyshev polynomials is assumed to build the temperature profile $T(\mathbf{x})$:

$$T(\mathbf{x}) = \sum_{k=1}^{K} t_k \cdot h_k(\mathbf{x}), \qquad \text{Eq. 3}$$

where the $h_k(\mathbf{x})$ are the Chebyshev polynomials, and the $t_k$ are the respective weighting coefficients. $K$ is the number of polynomials in the sum which is assumed to result in a good approximation of the real temperature profile $T(\mathbf{x})$ by eq. 3. Using eq. 3, eq. 1 can be rewritten in matrix form:

$$\mathbf{b} = \mathbf{A} \cdot \mathbf{t}, \qquad \text{Eq. 4}$$

with

$$A_{i,k} = \gamma_i \cdot \int_{\Omega} W_i(\mathbf{x}) \cdot h_k(\mathbf{x}) d\mathbf{x}.$$

This notation leads quite naturally to matrix inversion and Tikhonov regularization as used in **[2]** to solve the microwave reconstruction problem with Chebychev polynomials or potentially other basis functions. This approach though has still some important shortcomings which will be illuminated in the following paragraphs, and which motivated the disclosed invention.

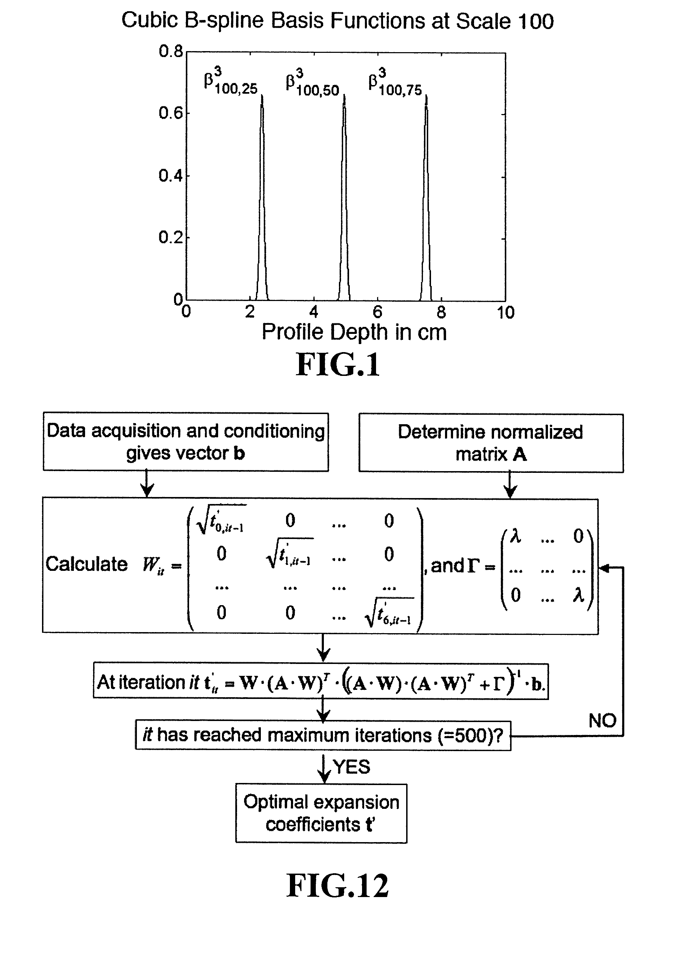

Let us shortly assume an example where the Chebychev polynomials of [2] are replaced by 100 B-splines of order 3 (cubic B-splines) at one single scale and covering the whole imaging depth. The sum of eq. 3 with 100 B-splines would be able to represent a very large variety of temperature profiles $T(\mathbf{x})$. In fig. 1, three of the 100 B-splines are drawn. Using all these 100 basis functions to reconstruct from the, let's say, 7 microwave measurements a temperature profile is not trivial, as one would try to solve a system of 7 equations for 100 unknowns $t_k$ (eq. 4 with **A** being a 7x100 matrix, i.e. an ill-posed problem). Such a system is heavily underdetermined, which means that a large or probably even infinite number of solutions exists. In **[2]**, Tikhonov regularization is proposed in order to deal with the ill-posed character of the resulting reconstruction problem. Other constrained reconstruction algorithms could be applied as well with the same motivations. They all have in common that they select between the large set of possible solutions the one that fulfills some imposed regularity constrained. Tikhonov regularization, for example, applies a smoothness constrained by imposing a minimal $l_2$-norm on the coefficients **t**, i.e. solving the following minimization problem in the least-square sense for **t**:

$$\min \lVert \mathbf{A} \cdot \mathbf{t} - \mathbf{b} \rVert + \lambda \cdot \mathbf{t} \cdot \mathbf{t}^T, \qquad\qquad \text{Eq. 5}$$

where $\lambda$ weights the importance of the smoothing constraint when solving eq. 1 for the optimal temperature profile, *T (x)*. On the contrary to eq. 4, eq. 5 is not an ill-posed problem, as long as $\lambda$ stays sufficiently big. The least-square solution to eq. 4 is written:

$$\mathbf{t} = (\mathbf{A}^T \cdot \mathbf{A} + \lambda \cdot \mathbf{I})^{-1} \cdot \mathbf{A}^T \cdot \mathbf{b}. \qquad\qquad \text{Eq. 6}$$

Here, **I** is the identity matrix. One can see that when $\lambda$ gets too small and the matrix $\mathbf{A}^T \mathbf{A}$ is ill-conditioned (which is in general the case for microwave temperature imaging) the inverse in eq. 6 does not exist.

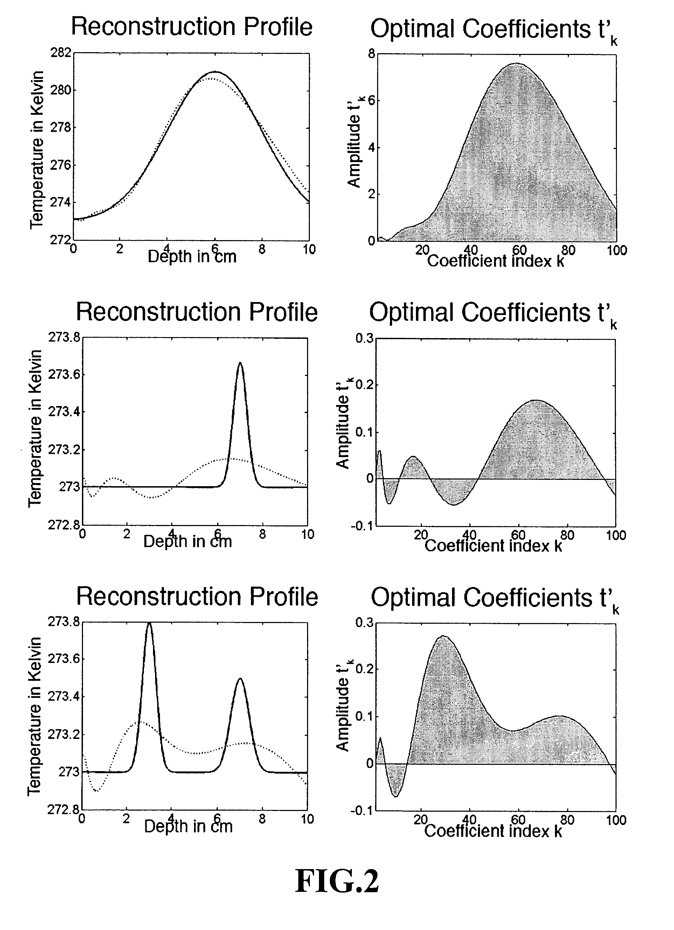

In fig. 2, we show some reconstruction results using the algorithm of [2] with 100 B-splines covering regularly the imaging space. The reconstruction results are represented in broken lines and the references in full lines. One sees easily that this approach does not always lead to satisfactory results. The regularization term over-smoothes the reconstruction result when the temperature profile to be recovered is not smooth enough itself. On the other, it's not possible to decrease the smoothing parameter, $\lambda$, beyond some minimum, as this would result again in inverting an ill-conditioned matrix $\mathbf{A}^T \cdot \mathbf{A} + \lambda \cdot \mathbf{I}$. On the right hand side of fig. 2, one can also observe that this state-of-the-art approach to profile reconstruction results in a very large weighted sum of B-spline function, as almost all coefficients **t** are significantly larger than zero. These arguments describe the motivations for the disclosed invention. They can be summarized as follows:

1) The reconstruction algorithm has to deal with the ill-posed character of the reconstruction problem, while being applicable to a wide range of possible real-world temperature profiles.
2) The algorithm must not be dominated by any regularization term provoking oversmoothing or other artifacts to appear.

The general solution to these problems as disclosed in this invention can be summarized as follows:

1) A large, over-complete dictionary of basis functions, such as B-splines at different scales and positions, have to be used in order to represent the variety of possible real-world temperature profiles with a small sum of these basis functions. The fact that a small sum of basis functions is sufficient for the reconstruction avoids oversmoothing of the resulting temperature profile estimation.
2) A regularized reconstruction algorithm has to be used which selects the best small set of basis functions out of the large set of initial basis functions (the over-complete dictionary) and which determines their weights to reconstruct the temperature profile. The regularization has to be as not to over-smooth the reconstruction result as shown to happen with e.g. Tikhonov regularization.

In conclusion, the disclosed invention describes a temperature image reconstruction procedure which, on the one hand, is capable to deal with the sparseness of the acquired radiometric data, and, on the other hand, succeeds in reconstructing a wide range of real-world temperature profiles for real-world medical and industrial applications without artifacts from the regularization term.

As will be shown in more detail in the following paragraphs, the disclosed invention maps the reconstruction problem into a well known formulation of decomposing signals over redundant dictionaries and uses standard algorithms, such as matching pursuit [3], orthogonal matching pursuit [4], basis pursuit [5], high-resolution matching pursuit [6], etc. to solve the resulting equivalent problem.

The disclosed invention applies equivalently to 1D (profiles), 2D (images), or 3D (volumes) temperature image reconstruction.

**Detailed Description of the Preferred Embodiments**

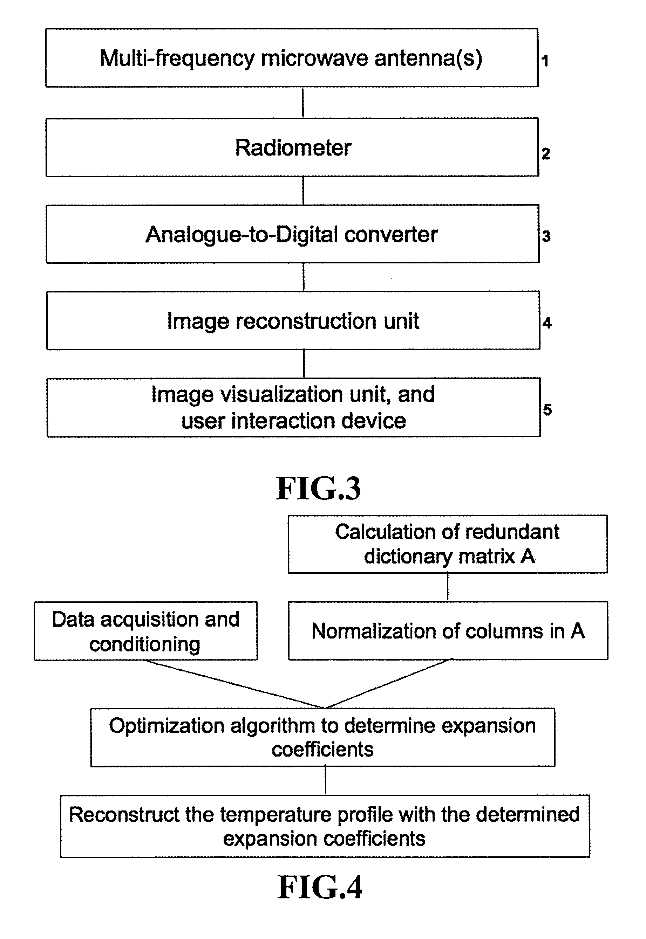

The disclosed invention for radiometric temperature image reconstruction has to be implemented on a completely functional microwave temperature imaging device. Several design implementations of such a system are possible, all of which share common characteristics though. In fig. 3, the functional building blocks common to all these systems are presented. First, we need one or several multi-frequency microwave antennas **1** for sensing the brightness temperatures, next a radiometer **2** has to interpret the signal and translate the sensed microwave radiation power into units of voltage. An analogue-to-digital converter **3** then digitizes the analogue voltage, so that the reconstruction unit **4**, basically any digital signal processing device, can reconstruct the intra-body temperature profile which caused the sensed microwave radiation. As final step, the temperature profiles are visualized for inspection by the medical doctor or industrial investigator on a visualization unit, such as a computer screen **5**. The disclosed invention is physically situated in the reconstruction unit and describes how temperature profiles can reliably be reconstructed from the sparse microwave radiation measurements. This reconstruction procedure will be described next.

Microwave image reconstruction aims to solve the following integral equation:

$$b(f) = \gamma_f \cdot \int_\Omega W(f, \mathbf{x}) \cdot T(\mathbf{x}) d\mathbf{x}, \qquad \text{Eq. 7}$$

where $b(f)$ is the measured brightness temperature sensed by the imaging antenna at frequency $f$, $\gamma_f$ is the antenna efficiency at frequency $f$, $W(f,\mathbf{x})$ is the spatial weighting function at frequency $f$, $\Omega$ is the sensing space of the microwave antenna, and $T(\mathbf{x})$ is the spatial temperature distribution the reconstruction algorithms aim to recover. The spatial variable x may be a 1D, 2D, or 3D vector, depending on the dimension of the reconstruction problem. Eq. 1 is known as a Fredholm equation of the first kind. There are several approaches to solving such equations [7]. In the concrete case of microwave temperature imaging, eq. 7 takes a slightly different form though. This is due to the fact that the brightness temperatures $b(f)$ can practically only be measured at a very restricted number of discrete frequencies, noted $f_i$, i=1,2,..., N, with N around 5 to 7 for the 1D case. This small number of measurements stands in contrast to the resolution of the temperature profile $T(\mathbf{x})$ one aims to obtain. Therefore, its practically impossible to discretize $T(\mathbf{x})$ to $T(\mathbf{x}_j)$, j=1,2, ...,M, where the $\mathbf{x}_j$ are the pixel coordinates, in order to find the matrix expression of eq. 1 as **b=W·t,** and using standard quadrature theory for the solution of eq. **1,** such as presented in [8]. This is because in the simplest 1D case, the number M would have already to lie at around 256 or 512 (number of pixels along the 1D profile) in order to reach a practically valuable resolution for the temperature profile $T(\mathbf{x})$. In other words, the matrix formulation would result in a system of only 5 to 7 linear equations with 256 or 512 unknowns $T(\mathbf{x}_j)$. Obviously this is a rather hard, if not impossible task to be done reliably, and in the case of a 2D or even 3D reconstruction, this problem would be become even harder.

Alternatively, and in contrast to the classical approach to solving Fredholm equations of the first kind, we reformulate eq. 7 in a semi-discrete fashion as follows:

$$b_i = \gamma_i \int_\Omega W_i(\mathbf{x}) \cdot T(\mathbf{x}) d\mathbf{x}, \text{ with } i = 1,2,...,N \qquad \text{Eq. 8}$$

where $i$ indexes the $N$ discrete measurement frequencies $f_i$ of the system. This formally quite simple change in problem formulation gives the possibility to solve the described image reconstruction problem for microwave radiometry in an attractive way, in particular for cases where the number of equations in eq8 is small in comparison to the resolution one would like to obtain for the temperature profile $T(\mathbf{x})$. In the following this approach is described in detail.

Intuitively, in order to solve eq 8 for the unknown function $T(\mathbf{x})$, one has to attempt to find a representation for the space of possible temperature profiles $T(\mathbf{x})$, which has to be entirely determined by not much more than the number of frequencies at which measurements are being carried out, while reflecting the large variability we might find in real-world temperature profiles. In addition, we have to propose algorithms which determine the best fit, i.e. the best function $T(\mathbf{x})$, for the given measurements $b_i$. The disclosed reconstruction procedure exactly presents a general approach to solve such a problem for microwave radiometry. In general, the disclosed approach to solve eq 8 for $T(\mathbf{x})$ can be structured into the following two steps:

1. Construct a function sub-space of $L_2$ (space of square- functions), denoted $\Omega_D$, spanned by a generally redundant set of functions, D={$h_k(\mathbf{x})$}, k=1,2,...,K, which accounts for the variability of possible temperature profiles T(x). This set has to be constructed in a way that for any temperature profile $T(\mathbf{x})$, which can realistically be expected, a small weighted sum of functions of D exists, which approximates the profile T(x). In this context, "small" means that the number of terms in the sum lies at around the number of measurements $N$ performed by the system:

$$T(\mathbf{x}) \approx \sum_{k=1}^{K} t_k \cdot h_k(\mathbf{x}), \qquad \text{Eq. 9}$$

with the majority of the coefficients $t_k$ being zero, and just about $N$ coefficients having significant magnitude.

2. Once such a set D has been constructed, for any given set of measurements $b_i$ an algorithm has to determine both, the functions of D and its weighting coefficients $t_k$ which approximate the temperature profile $T(\mathbf{x})$ best by the small weighted sum given in eq. 9.

The next paragraphs describe these two general steps in more detail.

**1. Over-complete set of basis functions for microwave radiometry**

Theoretically, any set of functions of $L_2$ would be admissible to span the reconstruction subspace of $L_2$, noted $\Omega_D$, and therefore constitute the basis set, D. Practically though, any prior information about the temperature profile $T(\mathbf{x})$ that can be incorporated into the chosen function set D should be employed in order to be able to implement an efficient and precise reconstruction algorithm. Furthermore, the basis set should be constructed, so that just a small sum of the potentially very large set of basis functions is able to reconstruct real-world temperature profiles $T(\mathbf{x})$ reliably. This means, that real-world temperature profiles $T(\mathbf{x})$ should have sparse representations in the space, $\Omega_D$.
Once the function set D has been constructed, one has still to implement an algorithm which is able to determine the small set of basis functions $h_k(\mathbf{x})$ and its associated weights $t_k$ which reconstruct an estimate of the unknown temperature profile T(x):

$$T(\mathbf{x}) \approx \sum_{k=1}^{K} t_k \cdot h_k(\mathbf{x}). \qquad \text{Eq. 10}$$

In mathematical terms, we need an algorithm which determines a sparse representation of the temperature profile $T(\mathbf{x})$ characterized by the set of measurements $b_i$ within the subspace $\Omega_D$ of $L_2$, spanned by the set of functions D. In the next paragraph such algorithms are shown.

**2. Reconstruction algorithm**

The initial reconstruction problem of eq. 7 can be rewritten, using the reconstruction approach described in the previous paragraph. Introducing eq. 10 into eq. 8, we get:

$$b_i = \sum_{k=1}^{K} t_k \cdot \gamma_i \cdot \int_{\Omega} W_i(\mathbf{x}) \cdot h_k(\mathbf{x}) d\mathbf{x}. \qquad \text{Eq. 11}$$

This problem formulation can also be written in matrix form as:

$$\mathbf{b} = \mathbf{A} \cdot \mathbf{t}, \qquad \text{Eq. 12}$$

with

$$A_{i,k} = \gamma_i \cdot \int_\Omega W_i(\mathbf{x}) \cdot h_k(\mathbf{x}) d\mathbf{x}. \qquad\qquad \text{Eq. 13}$$

The size of the matrix A is given by the number of measurements $N$ of the system, and by the number of functions $K$ in the redundant set D. The number of rows is therefore $N$, while the number of columns is $K$.

Eqs. 11 and 12 allow reinterpreting the temperature image reconstruction in a way which gives direct access to a large range of algorithms perfectly adapted to the problem of finding the optimal weighting coefficients $t_k$ for the profile reconstruction through eq. 9. In fact, eq. 13 can be seen as a transformation F, which transforms the space spanned by the basis functions of the set D, $\Omega_D$, onto a subspace of $\mathbf{R}^N$ (the vector space of $N$-dimensional vectors of real numbers), denoted $\Omega_R$:

$$\mathbf{F} : \Omega_D \subset L_2 \to \Omega_R \subseteq \mathbf{R}^N,$$

$$h_k(\mathbf{x}) \to \mathbf{a}_k = \int_\Omega \mathbf{W}(\mathbf{x}) \cdot h_k(\mathbf{x}) d\mathbf{x}, k = 1,2,...K,$$

$k = 1,2,...K,$
with

$$\mathbf{W}(\mathbf{x}) := \begin{pmatrix} \gamma_1 \cdot W_1(\mathbf{x}) \\ \gamma_2 \cdot W_2(\mathbf{x}) \\ \cdots\cdots \\ \gamma_N \cdot W_N(\mathbf{x}) \end{pmatrix}. \qquad\qquad \text{Eq. 14}$$

The resulting subspace of $\mathbf{R}^N$, $\Omega_R$, can be seen as a vector space spanned by the vectors $\{\mathbf{a}_k\}$ which are just the rows in the matrix $\mathbf{A}$ of eq. 12. As a last step, we normalize the vectors $\mathbf{a}_k$ and therefore the rows of A and reformulate the temperature profile reconstruction of eq. 12 accordingly:

$$\mathbf{b} = \sum_{k=1}^K t'_k \cdot \mathbf{a}'_k, \text{ with}$$

$$t'_k = t_k \cdot \|\mathbf{a}_k\|, \text{ and} \qquad\qquad \text{Eq. 15}$$

$$\mathbf{a}'_k = \frac{\mathbf{a}_k}{\|\mathbf{a}_k\|}.$$

The symbol $\|\mathbf{a}_k\|$ stands for the classical vector norm of $\mathbf{R}^N$ defined by

$$\|\mathbf{a}_k\| = \sqrt{\sum_i a_i^2}.$$

Eq. 15 is the reformulation of the initial temperature profile reconstruction, characterized by eq. 7, as standard sparse data decomposition over redundant dictionaries. Solving eq. 15 for a sparse representation aims to determine the few expansion coefficients $t'_k$ with significant magnitude, which result in a good approximation of **b**. Matching pursuit [3], orthogonal matching pursuit [4], basis pursuit [5], high-resolution matching pursuit [6], etc. are known algorithms for this task. Once this decomposition of **b** has been determined, the one-to-one correspondence of eq. 14 between the redundant set of base vectors of $\Omega_{\mathbf{R}}$, $\{a'_k\}$, and the redundant set of basis functions of $\Omega_{\mathbf{D}}$, $\{h_k(\mathbf{x})\}$, enables the temperature profile reconstruction through eq. 9:

$$T(\mathbf{x}) \approx \sum_{k=1}^{K} \frac{t'_k}{\|\mathbf{a}_k\|} \cdot h_k(\mathbf{x}). \qquad \text{Eq. 16}$$

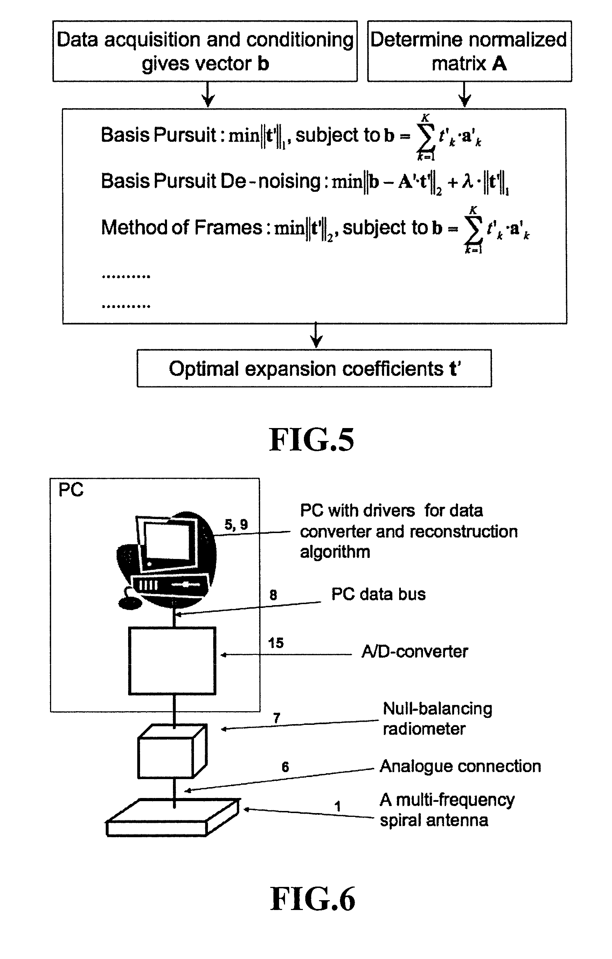

In fig. 4, a functional block-diagram is shown, which describes the different tasks that have to be implemented for the disclosed invention. In fig. 5, some algorithms that can be used in this disclosed invention are shown in relationship to its surrounding functional blocks. The mentioned algorithms represent not a succinct overview of possible algorithms which would solve eq. 15. Common to all of them is that they consist of two parts. First, they have the data part, which considers the actual measurements to determine the best temperature profile $T(\mathbf{x})$. And second, there is a sparseness constraint which ensures that just a small number of coefficients $t'_k$ has significant amplitude (sparse representation) and which, in the case of microwave temperature image reconstruction, plays the role of a regularization term that turns the generally ill-posed inversion problem of microwave temperature image reconstruction into a well-posed inversion problem. The difference of these "sparseness regularization terms" to the smoothness constraint of Tikhonov regularization (eq. 5), is that they do not over-smooth the final reconstruction result, $T(\mathbf{x})$. Let's shortly describe some of these well-known algorithms.

In the notational context of the previous paragraphs, general sparse decomposition over redundant dictionaries tries to solve the following equation:

$$\min \|\mathbf{t'}\|_0, \text{ subject to } \mathbf{b} = \mathbf{A'} \cdot \mathbf{t'}, \qquad \text{Eq. 17}$$

where $\|.\|_0$ denotes the $l_0$-norm. The minimization of the $l_0$-norm results in searching for a sparse representation, as the $l_0$-norm basically counts the non-zero expansion coefficients $t'_k$ in the expansion and tries to minimize them under the constrained that the data term is valid. Unfortunately, this problem can only be solved in a combinatorial way, and is therefore computationally very hard. Therefore one of the propositions consists of replacing the $l_0$-norm with a $l_1$-norm:

$$\min \|\mathbf{t'}\|_1, \text{ subject to } \mathbf{b} = \mathbf{A'} \cdot \mathbf{t'}. \qquad \text{Eq. 18}$$

where $\|.\|1$ denotes the $l_1$-norm. The fact of minimizing the $l_1$-norm of **t'** results in searching for a sparse representation, i.e. just few expansion coefficients $t'_k$ have significant magnitude. On the contrary to eq. 17, this problem can be solved with linear programming [5]. Interior point methods or simplex methods [13, 14, 15] can be used to solve eq. 18. Matlab's Optimization Toolbox [12, 15] has standard implementations of these algorithms which are perfectly suited to solve eq. 18.

A similar approach as basis pursuit is called basis pursuit denoising. It solves the following equation:

$$\min \|\mathbf{b} - \mathbf{A'} \cdot \mathbf{t'}\|_2 + \lambda \cdot \|\mathbf{t'}\|_1, \qquad \text{Eq. 19}$$

where $\lambda$ is a weighting coefficient, weighting the importance of the regularization term based on the $l_1$-norm of the decomposition coefficients, $t'_k$, and $\|.\|_2$ denotes the $l_2$-norm. The $l_1$-norm component of the minimization problem results in searching for a sparse representation of the initial reconstruction problem. This formulation of sparse data decomposition over redundant dictionaries can use quadratic programming [16, 17]. Again, this is computationally much more efficient than trying to solve eq. 17, and as for eq. 18, there is a standard implementation of quadratic optimization in Matlab's Optimization Toolbox [12].

As mentioned, a large number of further known algorithms exist, which can be applied to the presented invention, i.e. to sparse microwave temperature image reconstruction over redundant dictionaries. A complete overview of these algorithms would go beyond the scope of this text though. Common to all these algorithms is there localization within a concrete system design which would implement the disclosed invention, as was outlined in fig. 4 and 5.

**Detailed Description of further Embodiments**

As mentioned, the described reconstruction approach is applicable to 1D, 2D, or 3D reconstruction. The example implementations presented herein are in the context of the reconstruction of 1D temperature profiles for medical applications. Several medical applications could potentially take important profit from an easy-to-use and relatively cheap temperature measuring system. For example thermal tumor ablation is a medical procedure for cancer treatment whose success is directly related to the exact control of the intra-body temperature distribution. During a thermal tumor ablation treatment, thin needles are introduced percutaneously until their tips reach inside the tumor volume. At the tips, radiofrequency heats the tumor tissue up to a temperature guaranteeing cell death. In this procedure, two characteristics are fundamental for the success of the treatment: On the one hand, all the cancerous tissue has to be killed, and on the other hand, as few healthy tissue as possible should be ablated. As in such a treatment, the killing of the body cells is directly related to reaching or not reaching a particular temperature threshold, the specific system implementation presented herein can significantly improve the security and reliability of the medical treatment plan through real-time monitoring of the internal temperature distribution.

Tumor detection is mentioned as a second field of medical application for the presented system implementation. It employs the fact that tumor cells manifest themselves through a small local temperature increase of 1-2°C. Therefore, the temperature monitoring capability of the presented system allows the medical doctor to use temperature as an additional manifestation of cancerous cells during cancer screening.

Any concrete implementation of a microwave temperature imaging device has to contain a certain set of functional building blocks. These building blocks were sketched schematically in fig. 3. In the following, two concrete sample implementations of the general functional system are presented. Their main differences lie in the signal processing unit on which the images are reconstructed, and on the concrete reconstruction algorithm being employed for the reconstruction. Concretely, the first sample implementation uses an off-the-shelf personal computer (PC) as the signal processing unit for the image reconstruction, and the employed algorithm is a Matlab implementation of the basis pursuit algorithm as presented in **[5]**. The second implementation uses an embedded hardware device and the FOCUSS algorithm is used to find the basis pursuit de-noising solution to the reconstruction problem as presented in **[18]**.

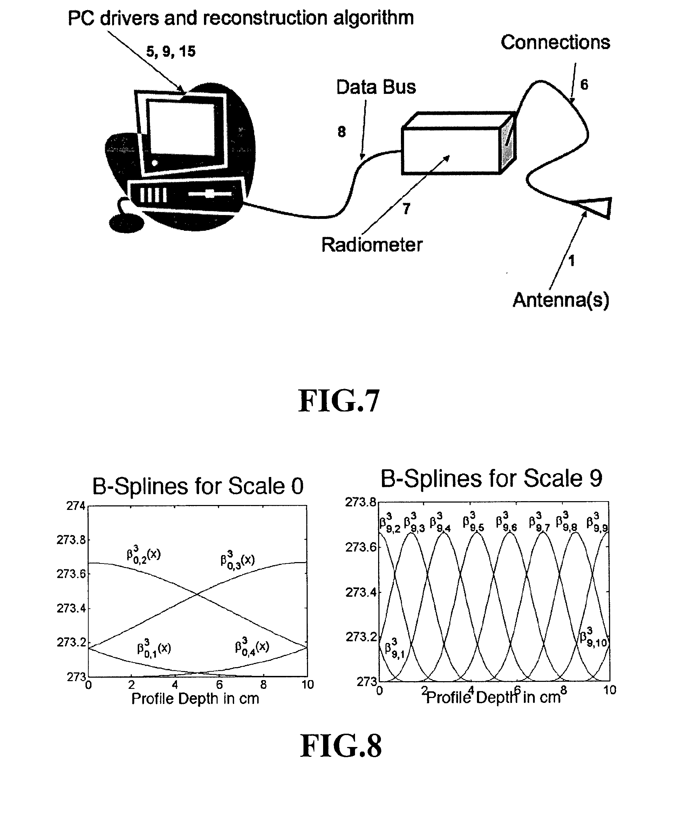

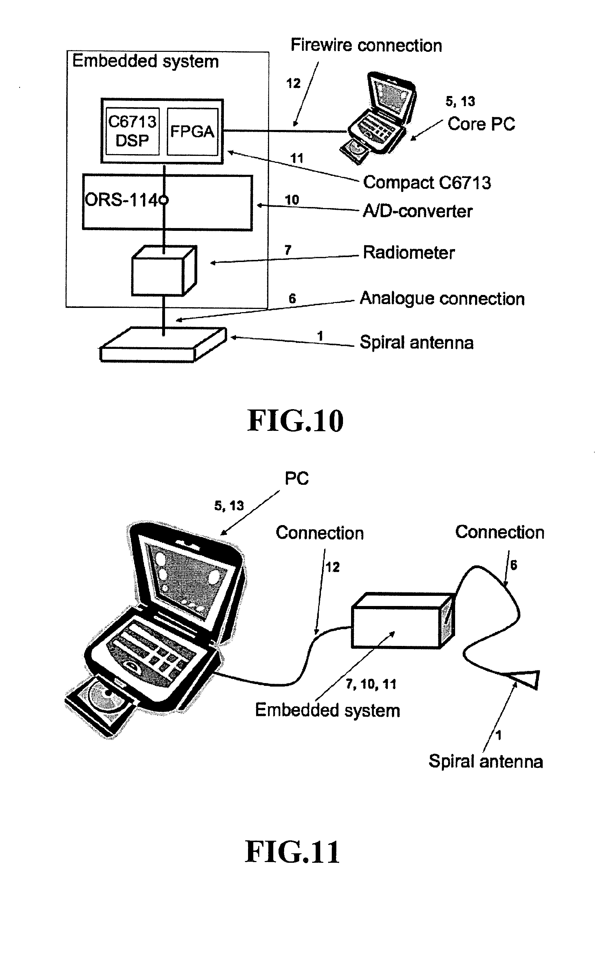

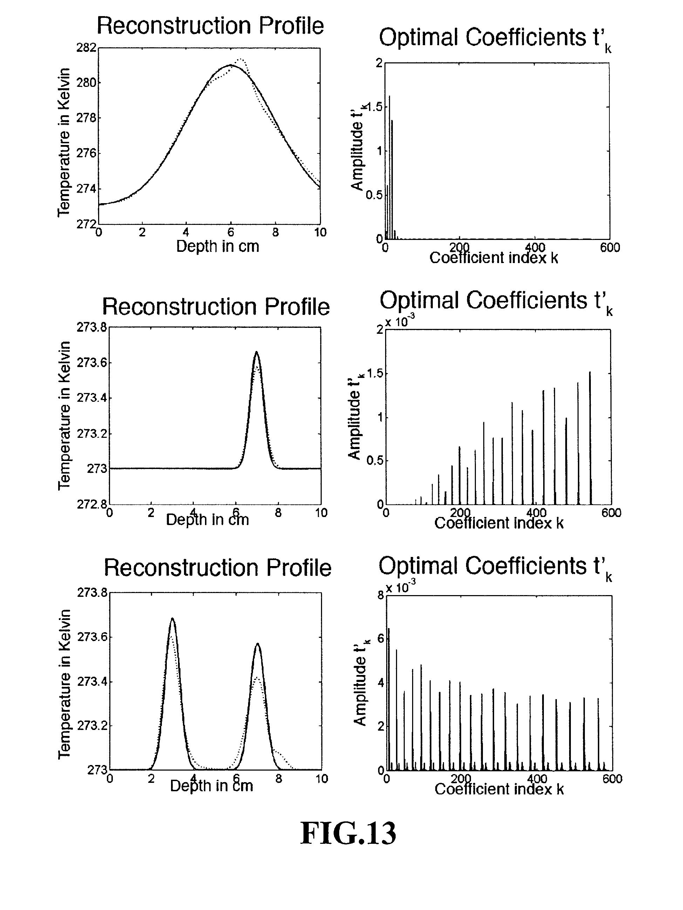

Fig. 6 and 7 illustrate a system comprising a multi-frequency spiral antenna or microwave spiral antennan(s) **1**, an analogue connection or connections **6**, a Dicke- null-balancing radiometer **7**, a PC data bus, such as USB, PCI, Fire Wire, or others **8** and a PC. The system according to fig. 6 comprises an off-the-shelf-PC **5, 9** with Matlab drivers for the analogue-to-digital data converter (Iotech DaqBoard/2000 Series) **15** and with the reconstruction algorithm implemented in Matlab. The system according to fig. 7 comprises a PC **5, 9, 15** with Iotech DaqBoard/2000 Series, corresponding Matlabs drivers and reconstruction algorithm implemented in Matlab. In fig. 6 and 7, the building blocks of the PC-based sample implementation are schematically shown, while the embedded sample implementation is drawn in fig. 11 and 12. Before presenting the implementation specific aspects of the devices, the common parts of both implementations are presented.

Both systems comprise one single (1D temperature profile) multi-frequency spiral microwave antenna **1** as disclosed by Jacobsen and Stauffer in **[9]**. The employed antenna operates at 7 frequencies in the range of 0.5 to 3.75 GHz. The signal sensed by the antenna passes through an analogue connection 6 to a Dicke null-balancing radiometer 7, as described by Jacobsen and al. in **[10]**. The Dicke radiometer detects sequentially the signal contributions at the different frequencies of the multi-frequency antenna of the system. The resulting sequential analogue signals from the radiometer are directly related to the brightness temperatures at the corresponding frequencies, and these brightness temperatures are directly related to the real intra-body temperature distribution through eq. 8. In order to reconstruct a one dimensional profile of real intra-body temperatures from the brightness temperatures, the reconstruction procedure disclosed herein is getting applied to the brightness temperatures, i.e. to the outputs from the analogue-to-digital converter **3, 10, 15**.

As mentioned in the general description, any specific reconstruction implementation needs to specify two aspects: first, the redundant set of base functions $\{h_k(\mathbf{x})\}$ has to be specified, and second, the optimization algorithm has to be chosen. It is important to note that in the present context of 1D temperature profile reconstruction, the spatial variable x parameterizes just a 1D space $\Omega$ i.e. is represented by one-component vectors. The size of the imaging space $\Omega$ is given by the maximal sensing depth $d_{max}$ of the microwave antenna, such as e.g. 10 cm for the cases presented herein.

In real-world medical applications for microwave temperature monitoring, such as cancer detection or thermal tumor ablation monitoring, bell-shaped temperature distributions can be expected. Therefore, we propose to use 1D B-splines at different scales and positions to build up the redundant set D of basis functions.

Herin, B-splines of order $N_d$=3 (cubic B-splines) and at $N_s$=30 consecutive scales are used to build the redundant set, D, of base functions. At the lowest scale, 4 B-splines cover the whole reconstruction space $\Omega$, while at each higher scale, one spline is added. Therefore, at each scale, s, $N_{p(s)}$=s+4 B-splines at different positions are added to the set, D, and the whole redundant set of basis functions can be parameterized as:

$$D = \{h_k(\mathbf{x})\} = \{\beta^3_{s,p(s)}(\mathbf{x})\},$$
$$s = 0,1,...,29,$$
$$p(s) = 1,2,...,s+4. \qquad\qquad \text{Eq. 20}$$

with

$$\beta^3_{s,p(s)}(x) = \begin{cases} \dfrac{2}{3} - |x|^2 + \dfrac{|x|^3}{2}, \text{if } 0 \leq \left| \left( x - d_{max} \cdot \dfrac{(p(s)-2)}{N_{p(s)}-3} \right) \cdot \dfrac{N_{p(s)}-3}{d_{max}} \right| \leq 1, \\[4mm] \dfrac{(2-|x|)^3}{6}, \text{if } 1 \leq \left| \left( x - d_{max} \cdot \dfrac{(p(s)-2)}{N_{p(s)}-3} \right) \cdot \dfrac{N_{p(s)}-3}{d_{max}} \right| \leq 2, \\[4mm] 0, \text{otherwise.} \end{cases}$$

Eq. 21

In fig. 8, the cubic B-splines basis functions for scale 0 and 9 are drawn in dependence on the profile depth in cm. The cardinality of D, $|D|=K$, is given by:

$$K = |D| = \sum_{s=0}^{N_S-1} N_{p(s)} = \sum_{s=0}^{N_S-1} s + 4 \qquad\qquad \text{Eq. 22}$$

Therefore in the present case, we have $|D|=K=555$. In accordance with the disclosed reconstruction procedure, we have to transform the base functions of D by the transformation given in eq. 14. In order to be able to do this, we need to specify the explicit weighting functions characterizing the used antenna. In the case of 1D reconstruction, an analytic model of the weighting function is given in **[2]**:

$$W_i(\mathbf{x}) = \frac{1}{d_i} \cdot e^{-x/d_i}, \qquad\qquad \text{Eq. 23}$$

where the parameters, $d_i$, are called the antenna sensing depths at frequencies $f_i$. For the employed spiral antenna and the chosen frequencies, the sensing depths lie at {0.9 cm, 1.2 cm, 1.5 cm, 1.8 cm, 2.1 cm, 2.4 cm, and 2.7 cm}. For this weighting function and for the chosen B-spline basis functions, an analytical solution for the transformation integral of eq. 14 exists, which can be determined easily using the following recursive formula:

$$\int x^n \cdot e^{ax} dx = \frac{1}{a} \cdot x^n \cdot e^{ax} - \frac{n}{a} \int x^{n-1} \cdot e^{ax} dx.$$

Eq. 24

This equation can be verified easily using partial integration, or it can be found in mathematical reference books such as in [23]. Therefore the vectors $\{a_k'\}$ can be calculated analytically. If the transformation integral of eq. 14 cannot be solved analytically, numerical integration has to be used.

The reconstruction process can formally be summarized for the sample implementations presented in this section as follows:

$$\mathbf{b} = \mathbf{A}'\cdot\mathbf{t}',$$

Eq. 25

where **b** is the vector whose components are the measured brightness temperatures at the 7 frequencies. The matrix **A'** is constituted of 555 normalized vector columns with 7 components each, noted $a_k'$, and t' are the 555 expansion coefficients one likes to recover. Therefore, eq. 13 becomes explicitely:

$$A_{i,k}' = \frac{A_{i,k}}{\|\mathbf{a}_k\|},$$

Eq. 26

$$A_{i,k} = \int_\Omega \frac{\gamma_i}{d_i} \cdot \exp\left(\frac{\mathbf{x}}{d_i}\right) \cdot \beta_k^3(\mathbf{x}) d\mathbf{x},$$

$i$ = 0,1,2,...,6 and $k$ =0,1,2,...,554.

Two concrete algorithms to solve eq. 25 for the unknowns **t'** are presented in the context of two concrete sample implementations in the following two sections. Once the parameters t' have been determined, the temperature profile $T(\mathbf{x})$ is calculated as

$$T(\mathbf{x}) = \sum_{k=0}^{554} \frac{t_k'}{\|\mathbf{a}_k\|} \cdot \beta_k^3(\mathbf{x}).$$

Eq. 27

The resulting reconstructed temperature profile T(**x**) is finally visualized on a computer screen 5 for visual inspection by the medical doctor or industrial investigator.

In the next two sections, the implementation specific aspects of the presented sample implementations will be presented.

**1. PC-based Sample Implementation**

The sample implementation outlined in fig. 6 and 7 is based on an off-the-shelf PC 9. It also comprises a PCI analogue-to-digital converter 15, such as Iotech's DaqBoard\2000 **[11]** with the provided Matlab **[12]** drivers, plugged in a PCI-slot **8** of the core PC. This converter converts the analogue signals from the Dicke null-balancing radiometer **7**, which represents the voltage encoded brightness temperatures, **b**, into their digital representations which are directly available inside the Matlab implementation of the reconstruction algorithm. This algorithm implements the general building blocks outlined in fig. 5, by calculating the matrix **A'** through eq. 26, and determining the expansion coefficients t' by basis pursuit **[5]**. Basis pursuit solves in the present context the following equation through the interior point algorithm already implemented in Matlab **[14, 15]**:

$$\min\lVert \mathbf{t'} \rVert_1, \text{subject to } \mathbf{b} = \mathbf{A'} \cdot \mathbf{t'}. \qquad \text{Eq. 28}$$

The resulting solution vector t' represents the expansion coefficients to be used for the temperature profile estimation by eq. 27.

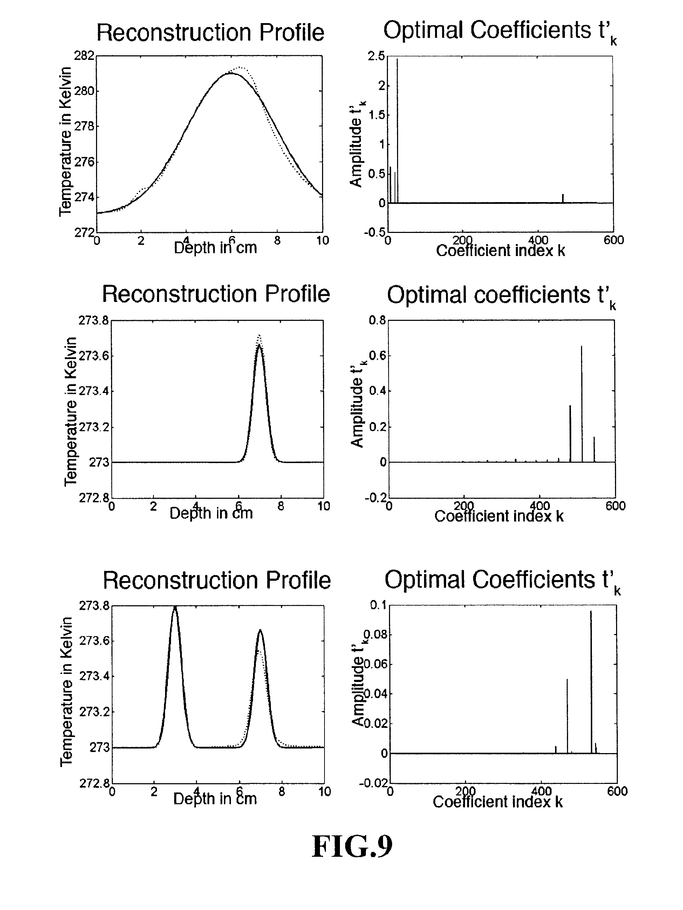

In fig. 9, three sample results are shown for the presented sample implementation, which used 100 iterations, and a convergence precision of $10^{-3}$ for the interior point algorithm of the Matlab Optimization Toolbox **[12].** The reconstruction results are represented in broken lines and the references in full lines. We see the clear advantage of the disclosed reconstruction approach compared to current state-of-the-art microwave temperature profile reconstruction shown in fig. 2.

## 2. Embedded Sample Implementation

Fig. 10 shows an embedded system for MW image reconstruction which is connected to a multifrequency spiral antenna **1** via an analogue connection 6 and comprises a dicke null-balancing radiometer **7**, an analogue-to-digital converter from Orsys, such as ORS-116, and the embedded development board C6713 Compact from Orsys connected via a firewire connection **12** to a core PC with Unibrain firewire drivers, wherein via said connection **12** images are sent to the core PC and configuration to the embedded system.

The system according to fig. **11** comprises a PC with firewire drivers and C++ API from Unibrain **5, 13,** a microwave spiral antenna 1 and an embedded system **7, 10, 11** for MW image reconstruction including a Dicke null-balancing radiometer, an analogue-to digital converter from Orsys and a C6713 Compact development board from Orsys. The embedded system **7, 10, 11** is connected to the PC **5, 13** via a firewire connection **12** and via an analogue connection **6** to the antenna **1**.

For the embedded sample implementation as outlined in fig. 10 and 11, the reconstruction algorithm is implemented on an embedded system **11** from Orsys **[21]**. More precisely, the radiometer **7** outputs, representing the voltage-encoded brightness temperatures sensed by the multi-frequency spiral antenna, are digitized by the ORS-116 data converter **10** from Orsys **[21]**. The resulting vector, **b,** with the brightness temperature components is being employed to reconstruct the temperature profiles on the C6713 Compact **11** from Orsys **[21].** The reconstruction result $T(\mathbf{x})$ is transmitted through the FireWire port **12** of the C6713 Compact device to the host computer **13**. On the host PC 13, the FireWire drivers by Unibrain **[22]** are being employed to receive the temperature profiles from the C6713 Compact device **11**. Finally, these temperature profiles are getting visualized on the computer screen **5** using vtk from Kitware **[19]** and Qt from Trolltech **[20].**

The reconstruction algorithm implemented on the embedded C6713 Compact device 11 from Orsys **[21]**, calculates the matrix **A'** through the mathematical expression given in eq. 26. The FOCUSS algorithm detailed in **[18]** is used to determine the expansion coefficients **t'** for the reconstruction of the temperature profile $T(\mathbf{x})$ through eq. 27. This algorithm solves the following expression iteratively as described in fig. 12:

$$\min\lVert \mathbf{b} - \mathbf{A'} \cdot \mathbf{t'} \rVert_2 + \lambda \cdot \lVert \mathbf{t'} \rVert_1. \qquad \text{Eq. 29}$$

Eq. 29 represents the so-called "basis pursuit de-noising" approach to data decomposition over redundant dictionaries. In fig. 13, the reconstruction results for this sample implementation are shown for 500 iterations, $\lambda=0.000001$, and where the initial estimate of $\mathbf{t'}_0$ ($it$=0) is a null vector. The reconstruction results are represented in broken lines and the references in full lines. The resulting coefficients **t'** determine the temperature profile $T(\mathbf{x})$ by the weighted sum of cubic B-splines as given in eq. 27.

## References

    **[1]** B. Stec, A. Dobrowolski, and W. Susek: "Multifrequency microwave thermography for biomedical applications"IEEE Transactions on Biomedical Engineering, Vol 51(3), March 2004

[2] S. Jacobsen and P.R. Stauffer: "Nonparametric 1-D temperature restoration in lossy media using Tikhonov regularization on sparse radiometry data", IEEE Transactions on Biomedical Engineering, Vol. 50(2), Feb. 2003

[3] S. Mallat and Z. Zhang: "Matching pursuit with time-frequency dictionaries", IEEE Transactions on Signal Processing, Vol. 41, Dec. 1993

[4] Y.C. Pati, R. Rezaiifar, and P.S. Krishnaprasad: "Orthogonal matching pursuits: recursive function approximation with applications to wavelet decomposition", Proceedings of the 27th Asilomar Conference in Signals, Systems, and Computers, 1993

[5] S.S. Chen, D.L. Donoho, and M.A. Saunders: "Atomic decomposition by basis pursuit", SIAM J. Sci. Comput., Vol 20(1), 1998

[6] S. Jaggi, W.C. Karl, St. Mallat, and A.S. Willsky: "High resolution pursuit for feature extraction", Technical Report, MIT, November 1996

[7] L.M. Delves, and J.L. Mohamed: "Computational methods for integral equations", Cambridge University Press, 1985

[8] W.H. Press, S.A. Teukolsky, W.T. Vetterling, and B.P. Flannery: "Numerical Recipes in C", Cambridge University Press, 1992 (Second Edition)

[9] S. Jacobsen and P.R. Stauffer: "Multifrequency Radiometric Determination of Temperature Profiles in a Lossy Homogeneous Phantom Using a Dual-Mode Antenna with Integral Water Bolus", IEEE Transactions on Microwave Theory and Techniques, vol. 50(7), pp. 1737-1746, July 2002.

[10] S. Jacobsen, P.R. Stauffer, and D. Neuman: "Dual-mode Antenna Design for Microwave Heating and Noninvasive Thermometry of Superficial Tissue Disease", IEEE Transactions on Biomedical Engineering, vol. 47, pp. 1500-1509, November 2000.

[11] http://www.iotech.com

[12] http://www.matlab.com

[13] G.B. Dantzig, A. Orden, and P. Wolfe: "Generalized Simplex Method for Minimizing a Linear from Under Linear Inequality Constraints", Pacific Journal Math, vol. 5, pp. 183-195.

[14] S. Mehrotra: "On the implementation of a Primal-Dual Interior Point Method",SIAM Journal on Optimization, vol. 2, pp. 575-601, 1992.

[15] Y. Zhang: "Solving Large-Scale Linear Programs by Interior-Point Methods under the MATLAB Environment", Technical Report TR96-01, Department of Mathematics and Statistics, University of Maryland, Baltimore County, Baltimore, MD, July 1995

[16] T.F. Coleman, and Y. Li: "A Reflective Newton Method for Minimizing a Quadratic Function Subject to Bounds on some of the Variables", SIAM Journal on Optimization, vol. 6(4), pp. 1040-1058, 1996.

[17] P.E. Gill, W. Murray, and M.H. Wright: "Practical Optimization" Academic Press, London, UK, 1981.

[18] B.D. Rao, K. Engan, S.F. Cotter, J. Palmer, and K. Kreutz-Delgado: "Subset Selection in Noise Based on Diversity Measure Minimization", IEEE Transactions on Signal Processing, vol 51(3), pp. 760-770, March 2003

[19] http://www.vtk.org

[20] http://www.trolltech.com

[21] http://www.orsys.de

[22] http://www.unibrain.com

[23] I.N. Bronstrein, K.A. Semendjajew, G. Musiol, and H. Mühlig "Taschenbuch der Mathematik" Verlag Harri Deutsch, 2nd edition, 1995

**Claims**

1. A microwave temperature image reconstruction system, which reconstructs from sensed microwave data one-, two- or three-dimensional temperature profiles or maps, which preferably uses known algorithms for solving data decomposition over redundant dictionaries in order to reconstruct the temperature profiles and maps, and/or which reconstructs the temperature profiles from very sparse sensing data for one-, two- or three-dimensional temperature profiles or maps.

2. System according to claim 1, having a reliable profile reconstruction approach applicable to industrial, medical, metrological or radiometric temperature measurement or monitoring applications.

3. System according to claim 1 or 2, comprising an over-complete dictionary of basis functions, such as B-splines, at different scales and positions in order to represent the variety of possible real-world temperature profiles with a small sum of these basis functions, and a regularized reconstruction algorithm which selects the best small set of basis functions out of the large set of initial basis functions or the over-complete dictionary and which determines their weights to reconstruct the temperature profile.

4. System according to one of the claims 1 to 3, for radiometric temperature image reconstruction, comprising one or several multi-frequency microwave antennas (1) for sensing the brightness temperatures, a radiometer (2) for interpreting the signal and translate the sensed microwave radiation power into units of voltage, an analogue-to- digital converter (3) for digitizing said analogue voltage, a reconstruction unit (4) for reconstructing the intra-body temperature profile which caused the sensed microwave radiation, and means for visualizing said temperature profiles, wherein the microwave image reconstruction is carried out by solving the following integral equation:

$$b(f) = \gamma_f \cdot \int_\Omega W(f, \mathbf{x}) \cdot T(\mathbf{x}) d\mathbf{x}, \qquad \text{Eq. A}$$

where $b(f)$ is the measured brightness temperature sensed by the imaging antenna at frequency $f$, $y_f$ is the antenna efficiency at frequency $f$, $W(f,\mathbf{x})$ is the spatial weighting function at frequency $f$, $\Omega$ is the sensing space of the microwave antenna, and $T(\mathbf{x})$ is the spatial temperature distribution the system aims to recover, and wherein the weighting function $W(f,\mathbf{x})$ weights the brightness temperature contributions from different spatial positions x.

5. System according to claim 4, wherein the equation A is reformulated as follows:

$$b_i = \gamma_i \int_\Omega W_i(\mathbf{x}) \cdot T(\mathbf{x}) d\mathbf{x}, \text{ with } i = 1,2,...,N \qquad \text{Eq. B}$$

where $i$ indexes the $N$ discrete measurement frequencies $f_i$ of the system.

6. System according to claim 5, wherein an approach to solve equation B for the spatial temperature distribution $T(\mathbf{x})$ is structured in the following two steps:

a) construct a function sub-space $\Omega_D$ of the space of square-integratable functions $L_2$, spanned by a generally redundant set of functions, D=$\{h_k(\mathbf{x})\}$, k=1,2,...,K, which accounts for the variability of possible temperature profiles $T(\mathbf{x})$, this set being constructed in a way that for any temperature profile $T(\mathbf{x})$, which can be expected, a small number of terms in a weighted sum of functions of D exists, which approximates the profile $T(\mathbf{x})$, said small number of terms in the sum corresponding to approximately the number of measurements $N$ performed by the system:

$$T(\mathbf{x}) \approx \sum_{k=1}^{K} t_k \cdot h_k(\mathbf{x}), \qquad\qquad \text{Eq. C}$$

with the majority of the coefficients $t_k$ being zero, and just about $N$ coefficients having significant magnitude, and b) once such a set D has been constructed, for any given set of measurements $b_i$ a reconstruction algorithm determines both, the functions of D and its weighting coefficients $t_k$ which approximate the temperature profile $T(\mathbf{x})$ best by the small weighted sum given in said equation C.

**7.** System according to one of the claims 4 to 6, wherein the equation A is reformulated as follows:

$$b_i = \sum_{k=1}^{K} t_k \cdot \gamma_i \cdot \int_{\Omega} W_i(\mathbf{x}) \cdot h_k(\mathbf{x}) dx. \qquad\qquad \text{Eq. D}$$

written in matrix form as:

$$\mathbf{b} = \mathbf{A} \cdot \mathbf{t}, \qquad\qquad \text{Eq. E}$$

with

$$A_{i,k} = \gamma_i \cdot \int_{\Omega} W_i(\mathbf{x}) \cdot h_k(\mathbf{x}) dx. \qquad\qquad \text{Eq. F}$$

wherein the size of the matrix A is given by the number of measurements $N$ of the system and by the number of functions K in the redundant set D, so that the number of rows is $N$ while the number of columns is K, wherein equation F is considered as a transformation F which transforms the space $\Omega_{\mathbf{D}}$ spanned by the basis functions of the set D onto a subspace of $\mathbf{R}^N$, denoted $\Omega_{\mathbf{R}}$, where $\mathbf{R}^N$ is the vector space of N-dimensional vectors of real numbers:

$$\mathsf{F} : \Omega_{\mathbf{D}} \subset L_2 \rightarrow \Omega_{\mathbf{R}} \subseteq \mathbf{R}^N,$$

$$h_k(\mathbf{x}) \rightarrow \mathbf{a}_k = \int_{\Omega} \mathbf{W}(\mathbf{x}) \cdot h_k(\mathbf{x}) dx, k = 1,2,...K,$$

with

$$\text{Eq. G}$$

$$\mathbf{W}(\mathbf{x}) := \begin{pmatrix} \gamma_1 \cdot W_1(\mathbf{x}) \\ \gamma_2 \cdot W_2(\mathbf{x}) \\ ...... \\ \gamma_N \cdot W_N(\mathbf{x}) \end{pmatrix}.$$

wherein the resulting subspace $\Omega_R$ of $\mathbf{R}^N$ is considered as a vector space spanned by the vectors $\{\mathbf{a}_k\}$ which are just the rows in the matrix $\mathbf{A}$ of eq. E, and including the step of normalizing the vectors $\mathbf{a}_k$ and therefore the rows $\mathbf{A}$, the temperature profile reconstruction of equation E can be reformulated as standard sparse data decomposition over redundant dictionaries as follows:

$$\mathbf{b} = \sum_{k=1}^{K} t'_k \cdot \mathbf{a}'_k, \text{ with}$$

$$t'_k = t_k \cdot \|\mathbf{a}_k\|, \text{ and} \qquad \text{Eq. H}$$

$$\mathbf{a}'_k = \frac{\mathbf{a}_k}{\|\mathbf{a}_k\|}.$$

where the symbol $\|\mathbf{a}_k\|$ stands for the classical vector norm of $\mathbf{R}^N$ defined by $\|\mathbf{a}_k\|^2 = \Sigma\, a_i^2$ and wherein therefore by solving equation H for a sparse representation the few expansion coefficients $t'_k$ with significant magnitude will be obtained.

8. System according to claim 7, wherein for solving Eq. H at least one of the following algorithms are used: matching pursuit, orthogonal matching pursuit, basis pursuit and/or high-resolution matching pursuit, or wherein algorithms are used to solve said Eq. H which consist of two parts, the data part, which considers the actual measurements to determine the best temperature profile $T(\mathbf{x})$ and a second part which ensures that just a small number of coefficients $t'_k$, has significant amplitude and which, in the case of microwave temperature image reconstruction, plays the role of a regularization term.

9. System according to one of the claims 1 to 8, comprising at least one multi-frequency spiral microwave antenna **(1)**, wherein the signal sensed by the antenna passes through an analogue connection **(6)** to a null-balancing radiometer **(7)** which detects sequentially the signal contributions at the different frequencies of the multi-frequency antenna of the system, the resulting sequential analogue signals from the radiometer being directly related to the brightness temperatures at the corresponding frequencies, and these brightness temperatures being directly related to a real intra-body temperature distribution, and comprising means to reconstruct a one dimensional profile of real intra-body temperatures from the brightness temperatures via the outputs from the analogue-to-digital converter **(3, 10, 15)**.

10. System according to claim 9, comprising means for specifying a redundant set of base functions $\{h_k(\mathbf{x})\}$ and means for optimizing a chosen algorithm for one-dimensional temperature profile reconstruction having a spatial variable $\mathbf{x}$, wherein said system parameterizes a one-dimensional space $\Omega$ and wherein the size of the imaging space $\Omega$ is given by the maximal sensing depth $d_{max}$ of the microwave antenna.

11. System according to claim 10, wherein B-splines of order $N_d$ and at $N_s$ consecutive scales are used to build the redundant set D of base functions, wherein at the lowest scale 4 B-splines cover the whole reconstruction space $\Omega$, while at each higher scale, one spline is added, so that at each scale $s$ a number of $N_{p(s)}=s+4$ B-splines at different positions are added to said set D, and wherein the whole redundant set of basis functions is parameterized as:

$$D = \{h_k(\mathbf{x})\} = \{\beta_{s,p(s)}^3(\mathbf{x})\},$$

$$s = 0,1,...,N_s,$$

$$p(s) = 1,2,...,s+4. \qquad \text{Eq. J}$$

with

$$\beta^3_{s,p(s)}(x) = \begin{cases} \dfrac{2}{3} - |x|^2 + \dfrac{|x|^3}{2}, \text{if } 0 \le \left| \left( x - d_{max} \cdot \dfrac{(p(s)-2)}{N_{p(s)} - 3} \right) \cdot \dfrac{N_{p(s)} - 3}{d_{max}} \right| \le 1, \\ \\ \dfrac{(2-|x|)^3}{6}, \text{if } 1 \le \left| \left( x - d_{max} \cdot \dfrac{(p(s)-2)}{N_{p(s)} - 3} \right) \cdot \dfrac{N_{p(s)} - 3}{d_{max}} \right| \le 2, \\ \\ 0, \text{otherwise.} \end{cases}$$

Eq. K

**12.** System according to one of the claims 1 to 11, comprising an off-the-shelf PC, a PCI analogue-to-digital converter **(15)**, preferable plugged in a PCI-slot **(8)** of the core PC, wherein said converter **(15)** converts the analogue signals from a null-balancing radiometer **(7)**, which represents the voltage encoded brightness temperatures **(b)** into their digital representations which are directly available inside the implementation of the reconstruction algorithm, wherein for the transformation integral of eq. G the following recursive formula is used

$$\int x^n \cdot e^{ax} dx = \frac{1}{a} \cdot x^n \cdot e^{ax} - \frac{n}{a} \int x^{n-1} \cdot e^{ax} dx.$$

Eq. L

and wherein the expansion coefficients **t'** of eq. L are determined by basis pursuit, wherein said basis pursuit solves the following equation through the interior point algorithm:

$$\min \| \mathbf{t'} \|_1, \text{subject to } \mathbf{b} = \mathbf{A'} \cdot \mathbf{t'}.$$

Eq. M

**13.** System according to one of the claims 1 to 12, including an embedded sample implementation calculating the Matrix **A'** through the mathematical expression given in the following equation

$$\min \| \mathbf{t'} \|_1, \text{subject to } \mathbf{b} = \mathbf{A'} \cdot \mathbf{t'}.$$

Eq. M

and solving the following expression iteratively

$$\min \| \mathbf{b} - \mathbf{A'} \cdot \mathbf{t'} \|_2 + \lambda \cdot \| \mathbf{t'} \|_1.$$

Eq. N

**14.** System according to one of the claims 1 to 13, comprising one single one-dimensional multi-frequency spiral microwave antenna operating at 7 frequencies in the range of 0.5 to 3.75 GHz, wherein the signal sensed by the antenna passes through an analogue connection to a Dicke null-balancing radiometer **(7)** which detects sequentially the signal contributions at the different frequencies of the multi-frequency antenna of the system.

## Cubic B-spline Basis Functions at Scale 100

FIG.1

FIG.12

**FIG.2**

Multi-frequency microwave antenna(s) 1

Radiometer 2

Analogue-to-Digital converter 3

Image reconstruction unit 4

Image visualization unit, and user interaction device 5

## FIG.3

Calculation of redundant dictionary matrix A

Data acquisition and conditioning

Normalization of columns in A

Optimization algorithm to determine expansion coefficients

Reconstruct the temperature profile with the determined expansion coefficients

## FIG.4

$$\text{Data acquisition and conditioning gives vector } \mathbf{b}$$

$$\text{Determine normalized matrix } \mathbf{A}$$

$$\text{Basis Pursuit}: \min\|\mathbf{t'}\|_1, \text{ subject to } \mathbf{b} = \sum_{k=1}^{K} t'_k \cdot \mathbf{a'}_k$$

$$\text{Basis Pursuit De-noising}: \min\|\mathbf{b} - \mathbf{A'} \cdot \mathbf{t'}\|_2 + \lambda \cdot \|\mathbf{t'}\|_1$$

$$\text{Method of Frames}: \min\|\mathbf{t'}\|_2, \text{ subject to } \mathbf{b} = \sum_{k=1}^{K} t'_k \cdot \mathbf{a'}_k$$

..........

..........

$$\text{Optimal expansion coefficients } \mathbf{t'}$$

## FIG.5

PC

PC with drivers for data converter and reconstruction algorithm — 5, 9

PC data bus — 8

A/D-converter — 15

Null-balancing radiometer — 7

Analogue connection — 6

A multi-frequency spiral antenna — 1

## FIG.6

22

PC drivers and reconstruction algorithm
5, 9, 15

Connections

Data Bus

6

8

Radiometer

7

1

Antenna(s)

# FIG.7

B-Splines for Scale 0

$\beta_{0,2}^3(x)$

$\beta_{0,3}^3(x)$

$\beta_{0,1}^3(x)$

$\beta_{0,4}^3(x)$

Profile Depth in cm

B-Splines for Scale 9

$\beta_{9,2}^3$ $\beta_{9,3}^3$ $\beta_{9,4}^3$ $\beta_{9,5}^3$ $\beta_{9,6}^3$ $\beta_{9,7}^3$ $\beta_{9,8}^3$ $\beta_{9,9}^3$

$\beta_{9,1}^3$

$\beta_{9,10}^3$

Profile Depth in cm

# FIG.8

FIG.9

Embedded system

Firewire connection

12

C6713 DSP | FPGA

5, 13
Core PC

11

Compact C6713

ORS-1140

10

A/D-converter

7

Radiometer

6

Analogue connection

1

Spiral antenna

## FIG.10

PC

5, 13

Connection

12

Connection

6

7, 10, 11

Embedded system

1

Spiral antenna

## FIG.11

**FIG.13**

**European Patent Office**

**EUROPEAN SEARCH REPORT**

Application Number

EP 05 40 5418

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | JACOBSEN S ET AL: "Nonparametric 1-D temperature restoration in lossy media using Tikhonov regularization on sparse radiometry data" IEEE TRANSACTIONS ON BIOMEDICAL ENGINEERING IEEE USA, vol. 50, no. 2, February 2003 (2003-02), pages 178-188, XP002379212 ISSN: 0018-9294 Sections II, IV, V. | 1-4,9,14 | INV. A61B5/00 G01K11/00 G01J5/60 |
| A | US 2003/088180 A1 (VAN VEEN BARRY D ET AL) 8 May 2003 (2003-05-08) * paragraphs [0005], [0032]; figure 3 * | 1,9,14 | |
| A | BAUSSARD ALEXANDRE ET AL: "Adaptive B-spline scheme for solving an inverse scattering problem" INVERSE PROBL; INVERSE PROBLEMS APRIL 2004, vol. 20, no. 2, April 2004 (2004-04), pages 347-365, XP002379214 Sections 1,4. | 1,11 | |
| A | KOLM R ET AL: "On the Discretization of Fredholm Integral Equations of First Kind Using Compactly Supported Wavelets" ANNU REV PROGR APPL COMPUT ELECTROMAGN; ANNUAL REVIEW OF PROGRESS IN APPLIED COMPUTATIONAL ELECTROMAGNETICS 2003, 2003, pages 339-346, XP008063738 * abstract * | 1,11 | TECHNICAL FIELDS SEARCHED (IPC) A61B G01K G01J |

-/--

The present search report has been drawn up for all claims

| Place of search | Date of completion of the search | Examiner |
|---|---|---|
| Berlin | 4 May 2006 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

# EP 1 738 683 A1

**European Patent Office**

## EUROPEAN SEARCH REPORT

**Application Number**

EP 05 40 5418

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GRIBONVAL R ET AL: "Approximation with highly redundant dictionaries signal processing applications" PROCEEDINGS OF THE SPIE - THE INTERNATIONAL SOCIETY FOR OPTICAL ENGINEERING SPIE-INT. SOC. OPT. ENG USA, vol. 5207, no. 1, 14 November 2003 (2003-11-14), pages 216-227, XP002379213 ISSN: 0277-786X * abstract * Sections 4.1-4.2 ----- | 1,3 | |
| A | PASTORINO M: "Recent inversion procedures for microwave imaging in biomedical, subsurface detection and nondestructive evaluation applications" MEASUREMENT, INSTITUTE OF MEASUREMENT AND CONTROL. LONDON, GB, vol. 36, no. 3-4, October 2004 (2004-10), pages 257-269, XP004633238 ISSN: 0263-2241 Sections 2.1, 2.4. ----- | 1 | **TECHNICAL FIELDS SEARCHED (IPC)** |

The present search report has been drawn up for all claims

| Place of search | Date of completion of the search | Examiner |
|---|---|---|
| Berlin | 4 May 2006 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 05 40 5418

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2006

| Patent document cited in search report | Publication date | Patent family member(s) | Publication date |
|---|---|---|---|
| US 2003088180 A1 | 08-05-2003 | NONE | |

For more details about this annex : see Official Journal of the European Patent Office, No. 12/82

# EP 1 738 683 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **B. STEC ; A. DOBROWOLSKI ; W. SUSEK.** Multi-frequency microwave thermography for biomedical applications. *IEEE Transactions on Biomedical Engineering,* March 2004, vol. 51
- **S. JACOBSEN ; P.R. STAUFFER.** Nonparametric 1-D temperature restoration in lossy media using Tikhonov regularization on sparse radiometry data. *IEEE Transactions on Biomedical Engineering,* February 2003, vol. 50 (2
- **S. MALLAT ; Z. ZHANG.** Matching pursuit with time-frequency dictionaries. *IEEE Transactions on Signal Processing,* December 1993, vol. 41
- **Y.C. PATI ; R. REZAIIFAR ; P.S. KRISHNAPRAS-AD.** Orthogonal matching pursuits: recursive function approximation with applications to wavelet decomposition. *Proceedings of the 27th Asilomar Conference in Signals, Systems, and Computers,* 1993
- **S.S. CHEN ; D.L. DONOHO ; M.A. SAUNDERS.** Atomic decomposition by basis pursuit. *SIAM J. Sci. Comput.,* 1998, vol. 20 (1
- **S. JAGGI ; W.C. KARL ; ST. MALLAT ; A.S. WILL-SKY.** High resolution pursuit for feature extraction. *Technical Report, MIT,* November 1996
- **L.M. DELVES ; J.L. MOHAMED.** Computational methods for integral equations. Cambridge University Press, 1985
- **W.H. PRESS ; S.A. TEUKOLSKY ; W.T. VETTERLING ; B.P. FLANNERY.** Numerical Recipes in C. Cambridge University Press, 1992
- **S. JACOBSEN ; P.R. STAUFFER.** Multifrequency Radiometric Determination of Temperature Profiles in a Lossy Homogeneous Phantom Using a Dual-Mode Antenna with Integral Water Bolus. *IEEE Transactions on Microwave Theory and Techniques,* July 2002, vol. 50 (7), 1737-1746

- **S. JACOBSEN ; P.R. STAUFFER ; D. NEUMAN.** Dual-mode Antenna Design for Microwave Heating and Noninvasive Thermometry of Superficial Tissue Disease. *IEEE Transactions on Biomedical Engineering,* November 2000, vol. 47, 1500-1509
- **G.B. DANTZIG ; A. ORDEN ; P. WOLFE.** Generalized Simplex Method for Minimizing a Linear from Under Linear Inequality Constraints. *Pacific Journal Math,* vol. 5, 183-195
- **S. MEHROTRA.** On the implementation of a Primal-Dual Interior Point Method. *SIAM Journal on Optimization,* 1992, vol. 2, 575-601
- **Y. ZHANG.** Solving Large-Scale Linear Programs by Interior-Point Methods under the MATLAB Environment. *Technical Report TR96-01, Department of Mathematics and Statistics,* July 1995
- **T.F. COLEMAN ; Y. LI.** A Reflective Newton Method for Minimizing a Quadratic Function Subject to Bounds on some of the Variables. *SIAM Journal on Optimization,* 1996, vol. 6 (4), 1040-1058
- **P.E. GILL ; W. MURRAY ; M.H. WRIGHT.** Practical Optimization. Academic Press, 1981
- **B.D. RAO ; K. ENGAN ; S.F. COTTER ; J. PALMER ; K. KREUTZ-DELGADO.** Subset Selection in Noise Based on Diversity Measure Minimization. *IEEE Transactions on Signal Processing,* March 2003, vol. 51 (3), 760-770
- **I.N. BRONSTREIN ; K.A. SEMENDJAJEW ; G. MUSIOL ; H. MÜHLIG.** Taschenbuch der Mathematik. Verlag Harri Deutsch, 1995